# EUROPEAN PATENT APPLICATION

(11) **EP 1 908 470 A1**
(43) Date of publication of application: **09.04.2008**
(21) Application number: 06756476.5
(22) Date of filing: 23.05.2006
(51) Int. Cl.: A61K 31/44, A61K 31/4415, A61K 31/675, A61P 7/08, A61M 1/28

(54) **PERITONEUM PROTECTING AGENT**

(30) Priority: 24.05.2005 JP 2005150887
(71) Applicant: TOKAI UNIVERSITY EDUCATIONAL SYSTEM, Shibuya-ku Tokyo 1510063 (JP)
(72) Inventor: KAKUTA, Takatoshi, c/o TOKAI UNIVERSITY, Isehara-shi, Kanagawa 259-1143 (JP); MIYATA, Toshio, c/o TOKAI UNIVERSITY, Isehara-shi, Kanagawa 259-1143 (JP)
(74) Representative: Polypatent
(86) International application number: PCT/JP2006/310220
(87) International publication number: WO 2006/126523

(57) **Abstract**

It is an objective of the present invention to provide a novel peritoneal membrane protecting agent which can effectively suppress deterioration of peritoneal functions in long-term peritoneal dialysis (PD) patients and the like. The present invention provides a peritoneal membrane protecting agent comprising a pyridoxine or a salt thereof, as an active ingredient.

## Description

### Technical Field

The present invention relates to a peritoneal membrane protecting agent comprising a pyridoxine such as pyridoxamine, as an active ingredient.

### Background Art

In long-term peritoneal dialysis (PD) patients, their peritoneal functions gradually deteriorate, which is characterized by an enhanced dissipation of the glucose-dependent osmotic gradient through the peritoneal membrane and a loss of the ultrafiltration ability [Non Patent Documents 1 and 2]. The best thing which describes such alterations in the functional characteristics of the failing peritoneal membrane is the increase in the functional area for exchanging small solutes (urea, creatinine, and glucose) between blood and dialysate (so-called "effective peritoneal surface area (EPSA)") [Non Patent Document 3]. Such alterations are due to enhanced angiogenesis, and probably, dilatation of peritoneal vessels [Non Patent Document 4].

The molecular mechanism providing a basis of the peritoneal dysfunction has not yet been elucidated. The recurrence of peritonitis along with inflammatory alterations damages the peritoneal membrane over a long term [Non Patent Documents 1 and 5], but is not a necessary condition for the onset of ultrafiltration failure [Non Patent Documents 4 and 6]. Recent studies have been presuming and revealing the mechanism of the deterioration of peritoneal functions in long-term PD patients [Non Patent Documents 7 to 14]. Chronic uremia itself alters the peritoneal membrane to increase EPSA [Non Patent Document 8]. Biochemical alterations in the peritoneal membrane are considered to be caused by, at least partially, overloading of extremely highly reactive carbonyl compounds (RCOs) originating from both uremic circulation and PD fluid ("peritoneal carbonyl stress") [Non Patent Documents 9 to 14]. During the uremic circulation, plasma RCOs are accumulated and slowly diffused in the peritoneal cavity to initiate the modification of advanced glycation end-products (AGEs) [Non Patent Document 15 to 21]. During PD, RCOs generated from the heat-sterilized glucose PD fluid invade the peritoneal membrane. RCOs are replenished by increasing the mass transfer of plasma RCOs by the dialysis treatment itself. This peritoneal carbonyl stress structurally promotes the irreversible AGE modification of peritoneal proteins. This RCO is believed to further stimulate the production of cytokines and growth factors (such as VEGF) to adjust the nitric oxide synthase (NOS) expression in a peritoneal cell [Non Patent Documents 22 to 25]. VEGF and nitric oxide (NO) jointly act to stimulate the angiogenesis, increase the permeability, and dilate the peritoneal capillaries [Non Patent Documents 23 to 25]. These composite alterations are considered to increase EPSA to thereby dissipate the osmotic pressure more quickly than normal, ending in unfeasible ultrafiltration. The upregulations of FGF-2 and TGF-β also stimulate the fibrosis of the peritoneal interstitium [Non Patent Documents 24 and 26].
[Non-patent document 1] Davies SJ,et al, Kidney Int 54: 2207-2217, 1998
[Non-patent document 2] Krediet RT, et al, Perit Dial Bull 6:61-65, 1986
[Non-patent document 3] Rippr B, et al, Peritoneal physiology: Transport of solutions. In: The Textbook of Peritoneal Dialysis, edited by Gokal R, Nolf KD, Dordrecht, The Netherlands, Kluwer Academic Publishers, 1994, 69-113
[Non-patent document 4] Krediet RT, et al, Kidney Int 55: 341-356, 1999
[Non-patent document 5] Krediet RT, et al, Ave Ren Replace Ther 5: 212-217, 1994
[Non-patent document 6] Struijk SG, et al,Kidney Int 45: 1739-1744, 1994
[Non-patent document 7] Davies SJ, et al,Nephrol Dial Transplant 11: 448-506, 1996
[Non-patent document 8] Combet S, et al,J Am Soc Nephrol 12: 2146-2157, 2001
[Non-patent document 9] Korbet SM, et al,Am J Kidney Dis 22: 588-591, 1993
[Non-patent document 10] Miyata T, et al,Kidney Int 2002; 61: 375-386
[Non-patent document 11] Nakayama M, et al,Kidney Int 51: 182-186, 1997
[Non-patent document 12] Miyata T, et al, Kidney Int 58: 425-435, 2000
[Non-patent document 13] Witowski J, et al,J Am Soc Nephrol 11: 729-739, 2000
[Non-patent document 14] Wieslander A, et al, Adv Perit Dial 12:57-60,1996
[Non-patent document 15] Garcia-Lopez E, et al,Perit Dial Int 20(Suppl 5): S48-S56, 2000
[Non-patent document 16] Krediet RT, et al,Perit Dial Int 17:35-41, 1997
[Non-patent document 17] Faller B, et al, Kidney Int (Supple 56): S81-S85, 1996
[Non-patent document 18] Rippe B, et al,Kidney Int 59:348-357, 2001
[Non-patent document 19] Topley N, Perit Dial Int 17:42-47, 1997
[Non-patent document 20] Feriani M, et al,Kidney Int 54: 1731-1738, 1998
[Non-patent document 21] Lage C, 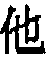 , Perit Dial Int 20(Suppl 5): S28-S32, 2000
[Non-patent document 22] Papapetropoulos A, et al, J Clin Invest 100: 3131-3139, 1997
[Non-patent document 23] Vriese AS, et al,J Am Soc Nephrol 12: 2029-2039, 2001
[Non-patent document 24] Margetts PJ, et al,J Am Soc Nephrol 12: 2029-39, 2001
[Non-patent document 25] Combet S, et al,J Am Soc Nephrol 11: 717-728, 2000
[Non-patent document 26] Ogata S, et al,J Am Soc Nephrol 12: 2787-2796, 2001

### Disclosure of the Invention

### Object to be Solved by the Invention

It is an objective of the present invention to provide a novel peritoneal membrane protecting agent which can effectively suppress deterioration of peritoneal functions in long-term peritoneal dialysis (PD) patients and the like.

### Means for Solving the Object

In order to verify the hypothesis that peritoneal dysfunctions and ultrafiltration failure in long-term peritoneal dialysis (PD) patients are associated with biochemical alterations in the peritoneal membrane which are induced by, at least partially, overloading of reactive carbonyl compounds (RCOs) originating from both uremic circulation and heat-sterilized glucose PD fluid ("peritoneal carbonyl stress"), the inventors of the present invention have conducted PD on subtotally nephrectomized uremic rat models using a typical glucose-based dialysate, to assess the protective effect of pyridoxamine (a recently-developed strong inhibitor against advanced glycation end-products (AGEs) and carbonyl stress) on structural, functional, and biochemical alterations in the peritoneal membrane. As a result, they have confirmed that the pyridoxamine exhibits the peritoneal protective effect, leading the completion of the present invention.

That is to say, according to the present invention, there is provided a peritoneal membrane protecting agent comprising a pyridoxine or a salt thereof, as an active ingredient.

Preferably, the pyridoxine is pyridoxamine.

Preferably, the peritoneal membrane protecting agent of the present invention is used for treating and/or preventing peritoneal dysfunction or ultrafiltration failure in a peritoneal dialysis patient.

According to another aspect of the present invention, there is provided a method for protecting the peritoneal membrane, comprising a step of administering an effective dose of a pyridoxine or a salt thereof to a mammal including human.

According to yet another aspect of the present invention, there is provided a use of a pyridoxine or a salt thereof for the production of a peritoneal membrane protecting agent.

### Best Mode for Carrying Out the Invention

Hereafter, embodiments of the present invention will be described in detail.

In the present invention, PD was conducted on subtotally nephrectomized uremic rat models using a typical glucose-based dialysate, to assess the protective effect of pyridoxamine (a recently-developed strong inhibitor against advanced glycation end-products (AGEs) and carbonyl stress) on structural, functional, and biochemical alterations in the peritoneal membrane.

Uremic peritoneal membranes have been characterized in increased functional area for exchanging small solutes between blood and dialysate, vascular proliferation, increased AGF production, and upregulations of the expression of angiogenic cytokines (namely, VEGF and FGF-2). PD-related structural, functional, and biochemical alterations in the peritoneal membrane show similar characteristics, the degree of which, however, are severer than those of chronic uremic patients without PD. The administration of pyridoxamine into uremic rats treated with PD significantly lowered the transport rate of small solutes and the vascular density. This result suggests beneficial roles of pyridoxamine with respect to the ultrafiltration failure. This improvement was accompanied by lowering in the AGE accumulation and the angiogenic cytokine expression.

In this manner, the peritoneal carbonyl stress caused by the uremic environment and the PD treatment serves as a causative factor of the vascular proliferation induced by bioactive molecules and the increased functional area for exchanging small solutes, finally leading to the ultrafiltration failure.

In the present invention, treatments using pyridoxamine significantly lowered the transport rate of small solutes from plasma, and the absorption rate of glucose from dialysate. Surprisingly, treatments using pyridoxamine almost completely dissipated the influence of uremia on the membrane permeability. This improvement of the peritoneal function was accompanied by the enhancement of angiogenesis induced by uremia and PD, and the suppression of the angiogenic cytokine expression. Actually, interventions using pyridoxamine significantly lowered the pentosidine content in tissues. Accordingly, pyridoxamine is capable of protecting the peritoneal membrane of uremic patients treated with PD, by improving functional, structural, and molecular biochemical alterations in the peritoneal membrane.

That is to say, the present invention relates to a peritoneal membrane protecting agent comprising a pyridoxine or a salt thereof, as an active ingredient.

Examples of the types of pyridoxines used as an active ingredient in the present invention include pyridoxine, pyridoxal, pyridoxamine, and salts thereof. Among the above, pyridoxamine or a salt thereof are preferably employed. The above pyridoxine, pyridoxal, and pyridoxamine refer to a chemical compound represented by the following formula: 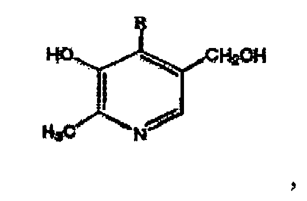 wherein R represents CH₂OH (in the case of pyridoxine), CHO (in the case of pyridoxal), or CH₂NH₂ (in the case of pyridoxamine).

Examples of the salts of pyridoxines include inorganic acid salts such as a hydrochloride, a sulfate, a nitrate, and a phosphate of the pyridoxine, or organic acid salts such as a maleate, a fumarate, a citrate, and an acetate thereof. Pyridoxine phosphate, pyridoxal phosphate, pyridoxamine phosphate, and the like are particularly preferred as such a salt.

Moreover, the pyridoxine or a salt thereof may be present in the form of a hydrate or a solvate. The pyridoxine or a salt thereof can be synthesized by a publicly known method, alternatively commercially purchased.

The peritoneal membrane protecting agent of the present invention comprises a pyridoxine as an active ingredient, and may be provided in the form of a pharmaceutical composition containing an additive for formulation, as desired. Such pharmaceutical compositions can be prepared by mixing the pyridoxine serving as an active ingredient in combination with a pharmaceutically acceptable carrier.

Examples of pharmaceutically acceptable carriers include, but are not limited to, physiological saline, Ringer's solution, phosphate-buffered physiological saline, and other carriers to that are already known by those skilled in the art. Such pharmaceutical compositions may contain one or more additives selected from a stabilizer, an antioxidant, a colorant, an excipient, a binder, a thickener, a dispersant, a reabsorption enhancer, a buffer, a surfactant, a preservative, an emulsifier, an isotonizing agent, a diluent, and the like, for example. Desirably, the abovementioned pharmaceutically acceptable carriers and additives are selected so as to minimize the side effects of the pyridoxine serving as an active ingredient and to lower the inhibition against the medicinal benefit of the pyridoxine. Preparation methods of pharmaceutical compositions comprising the pyridoxine in combination with a pharmaceutically acceptable carrier and/or an additive are already known by those skilled in the art.

For example, in cases where the peritoneal membrane protecting agent of the present invention is orally administered as a suspension, the pharmaceutical composition may be mixed with a microcrystalline cellulose, an alginic acid or sodium alginate as a suspending agent, a methylcellulose as a thickener, a fragrant material, and the like. In cases where immediate-release tablets are prepared, the pharmaceutical composition may be mixed with a microcrystalline cellulose, starch, magnesium stearate, lactose, or other excipients, a binder, an extender, a disintegrator, a diluent, a lubricant, and the like.

In cases where the peritoneal membrane protecting agent of the present invention is administered as an injectable solution or suspension, such an injectable solution or suspension can be prepared using an appropriate dispersant or humectant such as a sterilized oil containing synthetic mono- or di-glyceride and a fatty acid containing an oleic acid, and a suspending agent.

In cases where the peritoneal membrane protecting agent of the present invention is rectally administered as a suppository, such a suppository can be prepared by mixing the pyridoxine with an appropriate excipient such as a cocoa butter, synthetic glyceride ester, and polyethylene glycol, which is solid at normal temperatures, but is dissolved in the rectal cavity to release the drug.

The administration route of the peritoneal membrane protecting agent of the present invention is not specifically limited, and may be either one of oral administration and parenteral administration (such as subcutaneous administration, intramuscular administration, intracutaneous administration, and intravenous administration).

In cases where the peritoneal membrane protecting agent of the present invention is a solid preparation, the pyridoxine content in the solid preparation is normally 0.01 to 30 weight %, and preferably 0.1 to 20 weight %. In cases where the peritoneal membrane protecting agent of the present invention is a liquid preparation, the pyridoxine content in the liquid preparation is normally 0.1 to 20 mg/mL, and preferably 1 to 10 mg/mL.

The dose and the frequency of administration of the peritoneal membrane protecting agent of the present invention can be appropriately set according to various factors including the purpose of administration, the form of administration, the dosage form, and conditions such as the age, weight, and gender of the consumer. Typically, the dose is within a range of about 0.1 to 100 mg/kg, and preferably about 0.5 to 50 mg/kg per day, as a dose of the pyridoxine serving as an active ingredient. The formulation of the abovementioned dose is administered preferably once to four times a day. The administration period of the peritoneal membrane protecting agent of the present invention is not specifically limited, and the administration may be either in a short term (such as one day to several weeks) or a long term (several weeks to one month or more).

The peritoneal membrane protecting agent of the present invention can be used for treating and/or preventing peritoneal dysfunction or ultrafiltration failure in peritoneal dialysis patients. In the present specification, the term "treat" encompasses to heal and improve peritoneal dysfunction or ultrafiltration failure, or to prevent the progress thereof so as to relieve the exacerbation. Moreover, in the present specification, the term "prevent" encompasses to keep peritoneal dysfunction or ultrafiltration failure from occurring, or to restrain the exacerbation of the condition thereof.

In the present invention, the peritoneal membrane can be protected by administering the pyridoxine of an effective dose for treating or preventing peritoneal dysfunction or ultrafiltration failure to a patient (such as a peritoneal dialysis patient), by which the peritoneal dysfunction or ultrafiltration failure can be treated and/or prevented.

The present invention is hereafter described in greater detail with reference to the following example, although the present invention is not limited thereto.

### Example

### (A) Method

### (1) Experimental animal

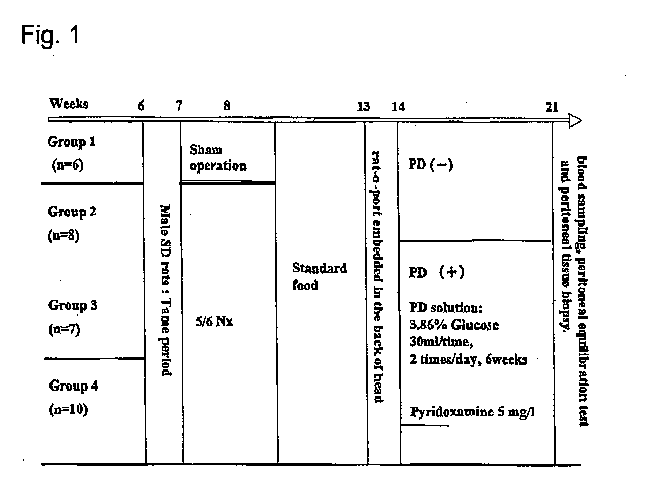

The protocol of the animal experiment is shown in Fig. 1. Six-week-old male Sprague-Dawley rats weighing 200±3 g (CLER Japan, Inc., Tokyo) were randomly assigned to: sham-operated control rats (Group 1: n = 6); uremic rats without peritoneal dialysis (Group 2: n = 12); and uremic rats undergoing peritoneal dialysis (n = 24). The uremic rats undergoing peritoneal dialysis were further divided into two groups: a group dialyzed with 4.25% Dianeal (registered trademark) (3.86% glucose, 2.5 mEq/CAPD fluid, Baxter Healthcare Corp., Round Lake, IL, USA) (Group 3: n = 12); and a group peritoneally dialyzed with 4.25% Dianeal (registered trademark) containing pyridoxamine (Pridorine, Biostratum Inc., NC, USA) at 5 mg/l (Group 4: n = 12).

Uremic models were generated by 5/6 nephrectomy according to previous reports [Lameire N, et al., Kidney Int 54: 2194-2206, 1998; and Kleinknecht C, et al., Kidney Int 47: S51-S54, 1995]. The animals were provided free access to standard experimental rat chow (CE2: CLER Japan, Inc., Tokyo) containing 1.06% calcium, 0.9% phosphate, and 21 % proteins, and tap water until the evening prior to slaughter. On the 13th week, Rat-o-Ports with a 13.5 cm catheter (Access Technologies, Norfolk Medical, Skokie, IL, USA) were implanted into all the rats under anesthetization of isoflurane. The Rat-o-ports were implanted subcutaneously in the neck, after one week of which the peritoneal dialysis with 30 ml of dialysate was initiated twice a day. The PD was continued for 6 weeks [Zweers MM, et al., Nephrol Dial Transplant 16: 651-654, 2001]. Before the initiation of the peritoneal dialysis, four, five, and two rats had died respectively in Group 2, Group 3, and Group 4. In the end, there were obtained: sham-operated control group (Group 1 : n = 6); uremic group without peritoneal dialysis (Group 2: n = 8); uremic group undergoing peritoneal dialysis (Group 3: n = 7); and uremic group undergoing peritoneal dialysis with 5 mg pyridoxamine (Group 4: n = 10).

### (2) Tissue collection

After 6 weeks of PD, 4.25% Dianeal (registered trademark) was injected from the access port and a peritoneal equilibration test (60 minutes) was performed. Rats were then euthanatized by overanesthetization. Plasma was separated from the heparinized blood obtained at the 21 st week. Mesenterium was excised and assessed by light microscopy or immunohistochemistry.

### (3) Analysis of plasma and peritoneal dialysate

The concentrations of glucose, creatinine, urea, total cholesterol, and triglyceride in plasma and peritoneal dialysate were measured with an autoanalyzer (Hitachi 736-60, Hitachi Co. Ltd., Tokyo).

### (4) Measurement of pentosidine with HPLC

The pentosidine content in the rat peritoneal membrane was assessed as follows, using HPLC in accordance with an already-reported method [Miyata T, et al., J. Am. Soc. Nephrol 7: 1198-1206, 1996]. The peritoneal tissue (100 mg) was homogenized with 1.5 ml of chloroform/methanol (2:1), and then was homogenized with 1.0 ml of methanol to remove lipid. The homogenized tissue was then dried under vacuum and hydrolyzed by 500 µl of 6N HCl at 110°C for 16 hours. Acid hydrolysates were dried in vacuo and reconstituted with 400 µl of 10 mM HCl, filtered through a 0.5 µm pore filter, and diluted with PBS. The diluted sample was injected into an HPLC system and separated on a C18 reverse-phase column (5 µm, 4.6 × 250 mm: Waters, Tokyo). The effluent was monitored using a fluorescence detector (RF-10A: Shimadzu, Tokyo) at an excitation-emission wavelength of 335/385 nm. Synthetic pentosidine was used to obtain a standard curve [Miyata T, et aL, J.Am. Soc. Nephrol 7: 1198-1206, 1996].

### (5) Immunohistochemistry

Immunohistochemistry was performed using formalin-fixed, paraffin-embedded sections. 4 µm sections were prepared from the paraffin block, followed by deparaffinization and dehydration by immersing in a series of ethanol concentrations. Primary antibodies used herein were anti-TGF-β1 antibody (1:40, Santa Cruz Biotechnology, Santa Cruz, CA, USA), anti-von Willebrand factor antibody (1:400, Daco, Glostrup, Denmark), anti-VEGF antibody (1:100, Santa Cruz Biotechnology), anti-FGF-2 antibody (1:400, Santa Cruz Biotechnology), and anti-AGE antibody (1:200) (Trans Genic Inc., Kumamoto). The sections were deparaffinized in xylene and dehydrated through ethanol. Endogenous peroxidase activity was inhibited by soaking the sections in 0.3% H₂O₂-containing methanol for 10 minutes at a room temperature. The heat-induced epitope retrieval was performed. After washing with phosphate buffered saline (pH 7.4), the sections (anti-TGF-β1, von Willebrand factor, FGF2, and VEGF antibody) were heated in 0.01M citrate buffer (pH 6.0) in a microwave oven (500W) for 10 minutes at 100°C. The sections were incubated with the primary antibodies for 1 hour at a room temperature. Then, the sections were rinsed and incubated with the secondary antibody (Nichirei Corp., Tokyo) for 45 minutes and were soaked in 0.2% 3, 3-diaminobenzidine tetrahydrochloride (DAB), followed by counterstaining with hematoxylin. The specificity of the immunolabeling was examined using nonimmune rabbit or goat IgG.

### (6) Assessment of number of vessels

The number of vessels was assessed in the sections stained for von Willebrand factor by image analysis using a WinROOF software (Mitani. Corporation, Tokyo). The average number of capillaries was roughly estimated with 20 fields of view that were randomly selected for each cortical region, using a 40x object lens. The results are expressed as the ratio of the number of vessels thereof to the number of vessels of healthy control animals.

### (7) Detection of VEGF and FGF-2 expressions

Expression of VEGF or FGF2 (mesenterium) mRNA was analyzed by using semiquantitative reverse transcriptase-polymerase chain reaction (RT-PCR). The total RNA was extracted from the mesenterium by using ISOGEN (Nippon Gene, Tokyo). 2 µg of the RNA was reverse transcribed using random primer (LIFE TECHNOLOGIES) with 200 units of MMLV reverse transcriptase (BioLabs. Inc.). PCR amplification was performed as already reported [Inagi R, et al. FEBS Letters 463: 260-264, 1999]. The sequences of oligonucleotide primers are as follows.
Primers for VEGF:
5'-ACT GGA CCC TGG CTT TAC TGC-3' (SEQ ID NO: 1)
5'-TTG GTG AGG TTT GAT CCG CAT G-3' (SEQ ID NO: 2)
The full-length amplified fragment is 310 bp long.
Primers for FGF2
5'-CAA GCA GAA GAG AGA GGA GTT-3' (SEQ ID NO: 3)
5'-TCAAGC TCT TAG CAG ACATTG-3' (SEQ ID NO: 4)
The full-length amplified fragment is 276 bp long.
Primers for rat β-actin
5'-GTG TGA TGG TGG GTA TGG GTC AGA AGG ACT-3' (SEQ ID NO: 5)
5'-ATG GCA TGA GGG AGC GCG TAA CCC TCA TAG-3' (SEQ ID NO: 6)
The full-length amplified fragment is 402 bp long.

β-actin was used as an internal standard of RNA to enable comparison of RNA levels among different samples. The samples were amplified in a DNA Thermal Cycler (Perkin Elmer Cetus, Norwalk, CT, USA) for suitable cycles of 1 minute at 94°C, 1 minute at 58°C for VEGF or FGF2, and 1 minute at 60°C and 1 minute at 72°C for β-actin. In the preliminary experiments, reverse transcription and PCR amplification were performed on various amounts of RNA for 18, 21, 25 28, 31, 34, and 36 cycles. These experiments revealed that, with 35 cycles of amplification for VEGF or FGF2 mRNA and with 16 or 21 cycles of amplification for β-actin, the differences in the PCR product signal were quantitatively related to the input RNA. The PCR products that were resolved by electrophoresis on a 2% agarose gel and stained using ethidium bromide were quantified by measuring the signal intensity with a quantitation program (NIH image software). The mRNA was determined in triplicate and the results were averaged for each experiment. A total of three to four independent experiments were performed for each experimental condition. The results were averaged and expressed as means ± SD.

### (8) Statistical analysis

Results were all expressed as means ± SD. The statistical significance of between-group differences was assessed by Wilcoxon nonparametric test (for comparing two groups) or Kruskal-Wallis test (for comparing three or more groups). In the statistical analysis of the present example, SPSS software (SPSS Inc., Chicago, IL, USA) was used to detect the significant difference. P-values less than 0.05 were regarded as significant.

### (B) Results

### (1) Functional Analysis

Table 1 shows biochemical data and body weight of subtotally nephrectomized uremic rats. The plasma creatinine, urea, and total cholesterol levels significantly increased and the body weight decreased in uremic rats (Groups 2 to 4) as compared with non-uremic rats (Group 1). Statistical analysis was performed between the presence/absence of PD and/or pyridoxamine treatment among the uremic groups, by which no significant difference was observed. The plasma concentrations of glucose and triglycerides were not statistically different across all groups.

**Table 1: Biochemical data and body weight (21 weeks)**

| Groups | Plasma creatinine (mg/gl) | Plasma urea (mg/dl) | Plasma glucose (mg/dl) | Triglycerides (mg/dl) | Total cholesterol (mg/dl) | Body weight (g) |
|---|---|---|---|---|---|---|
| 1 (n = 6) | 0.3±0.0 | 16.0±2.4 | 185.5±64.9 | 141.7±13.5 | 71.0±13,5 | 530±37 |
| 2 (n = 8) | 1.3±0.6* | 68.0±35.7 | 137.5±31.9 | 146.6±46.9 | 117.3±25.6* | 416±56* |
| 3 (n-7) | 1.6±0.3* | 62.8±13.0* | 121.1±36.3 | 157.1±58.4 | 143.1±33.9* | 435±10* |
| 4 (n =10) | 1.3±0.6* | 70.1±23.4* | 155.2±20.5 | 122.5±50.2 | 125.4±31.8* | 419±29* |

| | | | | | | |
|---|---|---|---|---|---|---|
| * : p<0.01 vs. Group 1. | | | | | | |

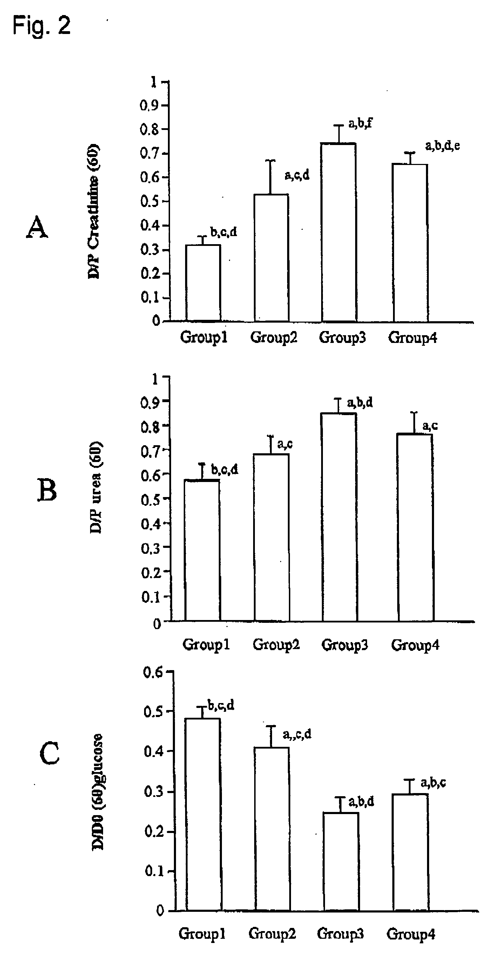

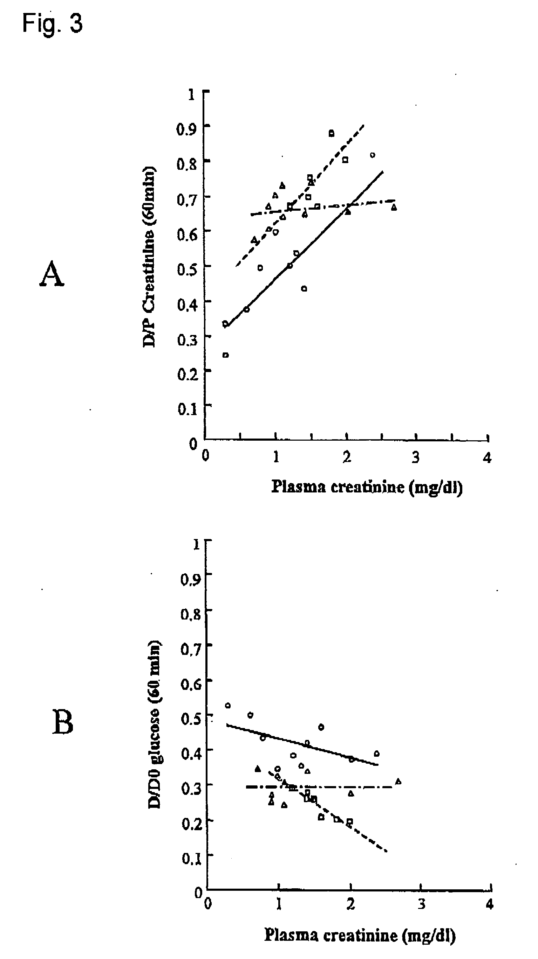

Functional parameters of the peritoneal membrane (PM) evaluated at the 13th week after subtotal nephrectomy showed that the transport rate of small solutes (i.e., creatinine and urea) from the plasma (figs. 2A and B) and the absorption rate of glucose from the dialysate (fig. 2C) were significantly higher in uremic rats (Group 2) than in control rats (Group 1). Significant correlations were observed between the increased permeability for creatinine (solid circle in fig. 3A) or glucose (fig. 3B) and the degree of renal failure. These data agree with recent observation results by Kombet *et al.* that chronic uremia by itself modifies the peripheral membrane and increases EPSA [Korbet SM, et al. Am J Kidney Dis 22: 588-591, 1993].

The permeability of peritoneal membrane permeability gradually alters during PD. The 6-week PD using a common glucose-based dialysate significantly increased the transport rate of small solutes from the plasma (Group 3 in figs. 2A and B) and the absorption rate of glucose from the dialysate (fig. 2C).

Alterations in the peritoneal membrane function due to uremia and PD treatment are considered to be caused by, at least partially, peritoneal carbonyl stress originating from both uremic circulation and PD fluid. Therefore, uremic rats were subjected to intraperitoneal PD using pyridoxamine (which is clinically used as strong inhibitor against AGEs and carbonyl stress). The 6-week treatment with pyridoxamine (5 mg/L) significantly lowered the transport rate of small solutes from the plasma (Group 4 in figs. 2A and B) and the absorption rate of glucose from the dialysate (fig. 2C). As shown in figs. 3A and B (open triangle), pyridoxamine dramatically lowered the slope of the regression line between the increased permeability for the small solutes (creatinine and glucose) and the degree of renal failure. Pyridoxamine treatment completely dissipated the influence of uremia on membrane permeability.

### (2) Histological analysis

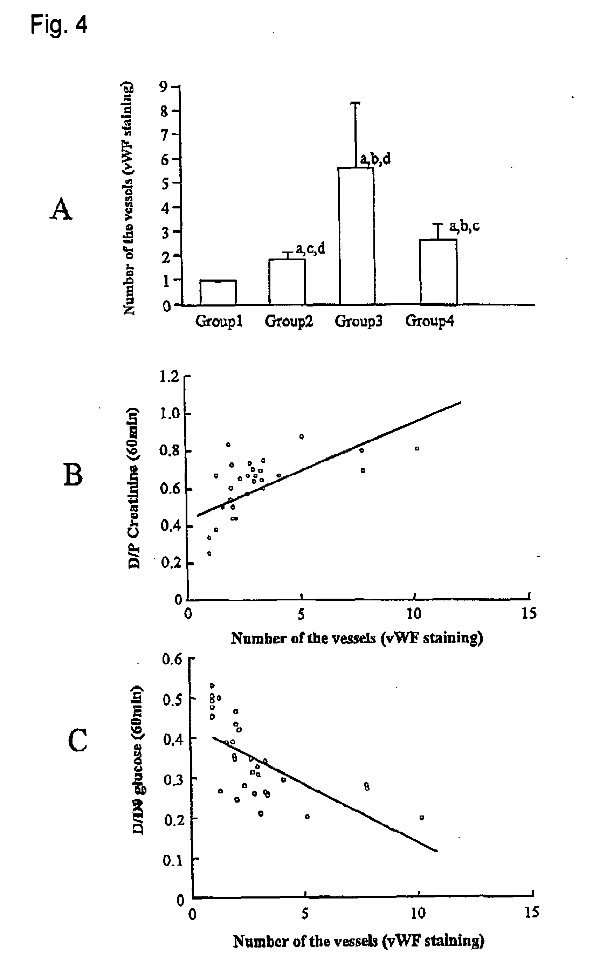

An increased EPSA is known to be closely related to enhanced angiogenesis. Therefore, the number of vessels in the peritoneal tissue after von Willebrand factor staining were counted (fig. 4A). Uremia by itself caused a 1.87-fold increase in the vessel number, and the PD treatment augmented the value by 5.83-fold. This enhanced angiogenesis was improved by the pyridoxamine treatment significantly to 2.68-fold. Significant correlations were observed between the increased permeability for creatinine (fig. 4B) or glucose (fig. 4C) and the number of vessels. This result means that the increased EPSA at the time of deterioration of the peritoneal membrane function is related to uremia and PD treatment.

### (3) Molecular biological analysis

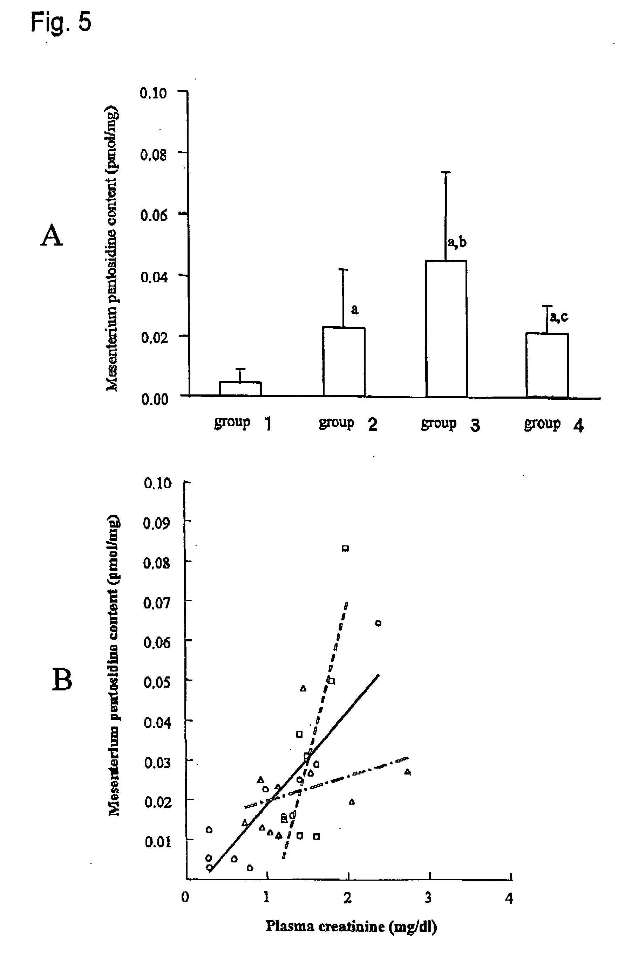

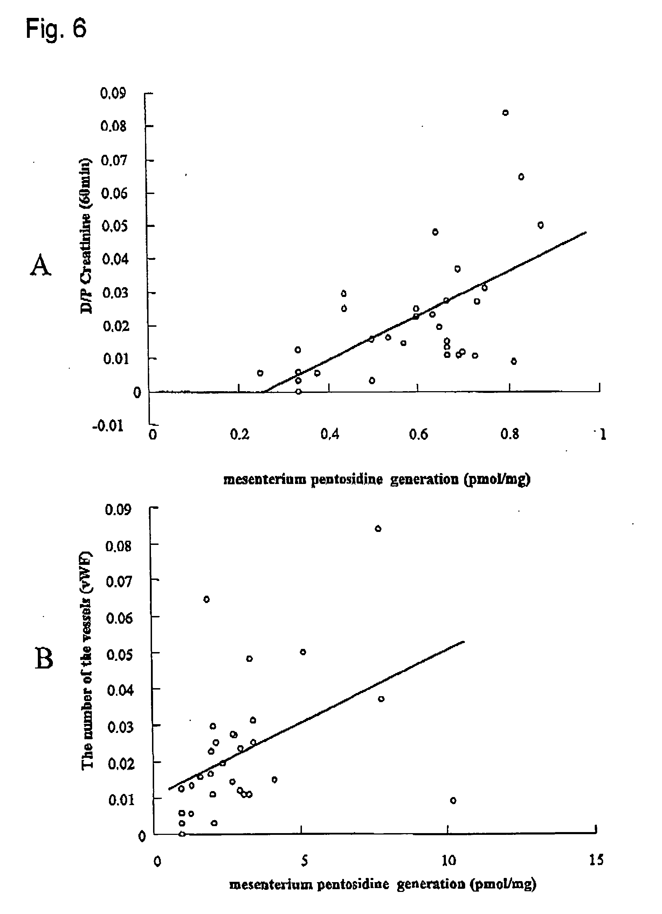

Molecular events which are associated with functional and structural alterations in the peritoneal membrane were examined. The content of pentosidine, serving as a publicly known alternative marker for AGE and carbonyl stress, in the peritoneal membrane was measured by HPLC assay. Pentosidine content increased during uremia (Group 2 in fig. 5A), and a correlation existed between the tissue pentosidine content and the degree of renal failure (solid circle in fig. 5B). The formation of pentosidine was accelerated during the PD treatment (Group 3 in fig. 5A), and PD increased the slope of the regression line between the pentosidine content and the renal failure (open square in fig. 5B). In contrast, intervention by pyridoxamine significantly lowered the tissue pentosidine content (Group 4 in fig. 5A) and lowered the slope of the regression line (open triangle in fig. 5B). Significant correlations were observed between the tissue pentosidine content and the increased permeability for creatinine (fig. 6A) and the number of vessels (fig. 6B).

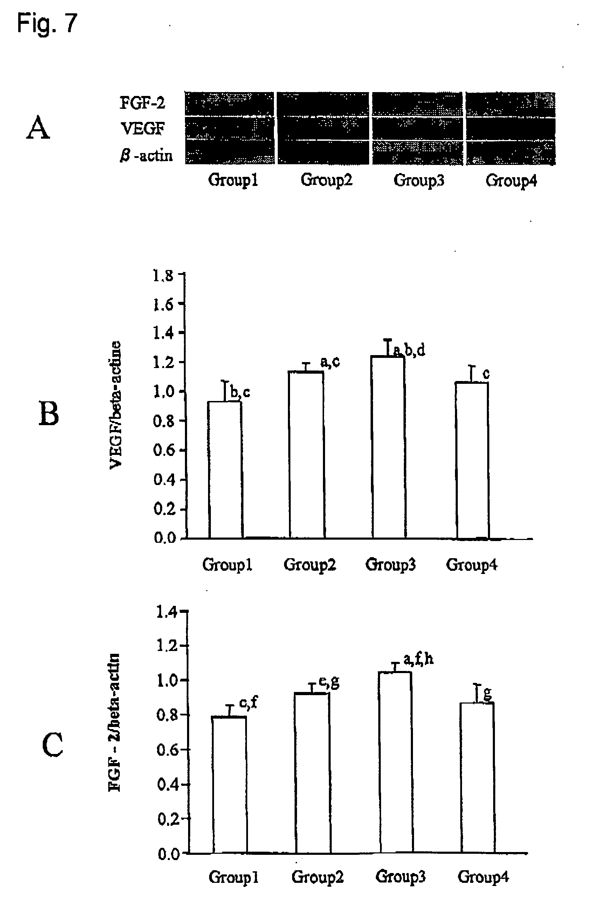

Next, in order to elucidate the roles of angiogenic cytokines, gene expressions of VEGF or FGF-2 in the peritoneal tissue were analyzed by semiquantitative PCR. The expressions of VEGF (fig. 7B) and FGF2 (fig. 7C) were higher in uremic rats than control rats, and were significantly increased by PD treatment. On the other hand, pyridoxamine lowered the both expressions of VEGF and FGF-2 to equivalent levels to those of uremic rats without PD.

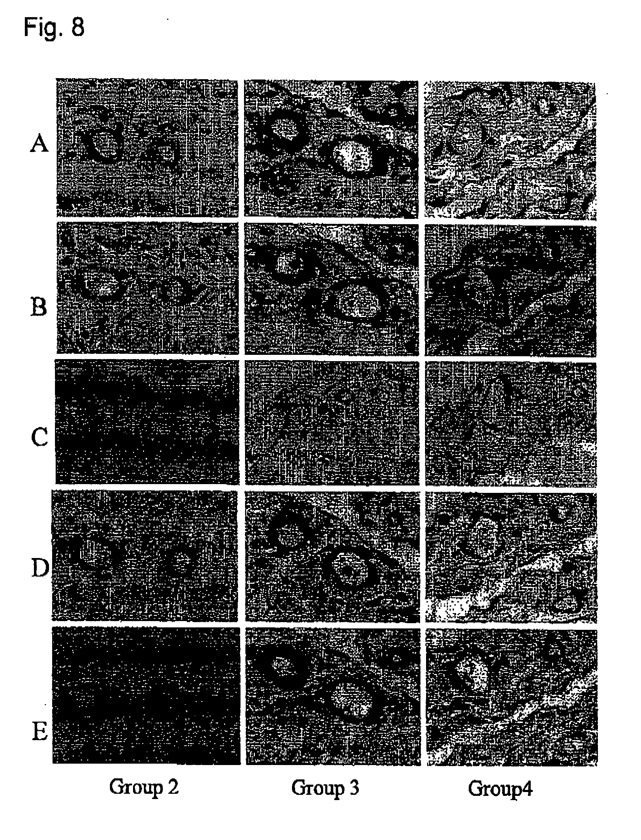

Molecular biological alterations in the peritoneal membrane were further supported by immunohistochemical analysis on VEGF, FGF-2, vWF, AGEs, and TGF-β1 in the sections of mesenterium tissue (fig. 8). That is to say, VEGF, FGF-2, vWF, AGEs, and TGF-β1 stainings were virtually undetectable in the vascular endothelium of mesenterium of control rats, whereas these biomarkers were faintly present in the peritoneal membrane of uremic rats and were prominent in uremic rats with PD treatment. On the other hand, only faint stainings were detected in the capillaries of the mesenterium in rats treated with pyridoxamine.

### Industrial Applicability

Pyridoxines such as pyridoxamine used as an active ingredient in the present invention are safe substances with less side effects. The present invention has enabled to provide safe peritoneal membrane protecting agent which can effectively suppress deterioration of peritoneal functions in long-term peritoneal dialysis (PD) patients, and the like.

### Brief Description of the Drawings

Fig. 1 shows a protocol for animal experiments. Six-week-old rats were assigned to uremic sham-operated groups. Five weeks after subtotal nephrectomy, intraperitoneal catheters and subcutaneous access ports were surgically implanted. PD was continued until the 21 st week after birth.
Fig. 2 shows a functional analysis of peritoneal membrane by PET. The dialysate/plasma (D/P) ratio of Cr (A) and urea (B), and the absorption of glucose from the dialysate (D/D₀) (C) were assessed, during a 60-minute exchange with 20 ml of 3.85% glucose dialysate, in control rats (Group 1), uremic rats without PD (Group 2), uremic rats undergoing peritoneal dialysis in the absence of pyridoxamine (Group 3), and uremic rats undergoing peritoneal dialysis in the presence of 5 mg/L pyridoxamine (Group 4). a: p<0.01 vs. Group 1. b: p<0.01 vs. Group 2. c: p<0.01 vs. Group 3. d: p<0.01 vs. Group 4. e: p<0.05 vs. Group 3. f: p<0.05 vs. Group 4.
Fig. 3 shows correlations between the increased permeability for creatinine (A) or glucose (B) and the degree of renal failure.
   Solid circle: Group 1 and Group 2 (A: y = 0.20x + 0.26, n = 14, r = 0.87, p< 0.001. B: y = 0.0538x + 0.49, n = 14, r = 0.63, p<0.05)
   Open square: Group 3 (A: y = 0.22x + 0.39, n = 7, r = 0.76, p>0.01. B: y = 0.1350x + 0.45, n = 7, r = 0.93, p<0.05)
   Open triangle: Group 4 (A: y = 0.02x + 0.63, n = 10, r = 0.24, p>0.1. B: y = 0.0015x + 0.29, n = 10, r = 0.025, p>0.1)
   Chronic uremia increases the permeability for small solutes, which is further increased by PD with common glucose-based dialysate. In contrast, pyridoxamine treatment keeps the membrane permeability from decreasing in uremia.
Fig. 4 shows structural analysis of peritoneal membrane by immunohistochemistry for von Willebrand factor.
   A: Number of vessels in the mesenterium of rats. a: p<0.01 vs. Group 1. b: p<0.01 vs. Group 2. c: p<0.01 vs. Group 3. d: p<0.01 vs. Group 4.
      Significant correlations existed between the increased permeability for creatinine (B: y = 0.052x + 0.434, n = 31, r = 0.65, p<0.001) or glucose (C: y = -0.029x + 0.43, n = 31, r = -0.64, p<0.001) and the number of vessels. Chronic uremia increases the number of vessels, which is further increased by PD with common glucose-based dialysate, but is decreased by pyridoxamine treatment.
Fig. 5 shows AGE content of peritoneal membrane by HPLC assay.
   A: Pentosidine content in the mesenterium of rats. a: p<0.01 vs. Group 1. b: p<0.05 vs. Group 4. c: p<0.05 vs. Group 3.
   B: A significant correlation exists between mesenterium pentosidine content and the degree of permeability for creatinine due to renal failure.
      Solid circle: Group 1 and Group 2 (y = 0.0.24x - 0.005, n = 14, r = 0.91, p<0.001)
      Open square: Group 3 (y = 0.80x + 0.091, n = 7, r = 0.82, p<0.01)
      Open triangle: Group 4 (y = 0.006x + 0.0135, n = 10, r = 0.35, p>0.1)
      Chronic uremia increases the mesenterium pentosidine content, which is further increased by PD with common glucose-based dialysate, but is decreased by pyridoxamine administration.
Fig. 6 shows correlations between mesenterium pentosidine content and the permeability for creatinine or the number of vessels. Mesenterium pentosidine content correlates with the permeability for creatinine (A: y = 0.066x - 0.018, n = 31, r = 0.65, p<0.001) or the number of vessels (B: y = 0.004x + 0.001, n = 31, r = 0.46, p<0.01).
Fig. 7 shows the detection of angiogenic cytokine expression. The expressions of VEGF or FGF2 mRNAs in the mesenterium were analyzed by semiquantitative reverse transcriptase-polymerase chain reaction (RT-PCR) (A). Mean ratio of VEGF or FGF2 mRNAs over β-actin was calculated for each experiment. The average of the two experiments is expressed for VEGF (B) or FGF2 (C). a: p<0.05 vs. Group 1. b: p<0.05 vs. Group 2. c: p<0.05 vs. Group 3. d: p<0.05 vs. Group 4. e: p<0.01 vs. Group 1. f: p<0.01 vs. Group 2. g: p<0.01 vs. Group 3. h: p<0.01 vs. Group 4.
Fig. 8 shows immunohistochemical detections of VEGF, FGF2, vWF, AGES, and TGFβ1 in the mesenterium. VEGF (A), FGF2 (B), vWF (C), AGEs (D), and TGF-β1 (D) stainings were virtually undetectable in the peritoneal membrane of control rats, whereas these biomarkers were faintly present in uremic rats and were prominent in uremic rats with PD treatment. In contrast, only faint stainings were detected in the capillaries of the mesenterium in rats administered with pyridoxamine (magnification x400).

## Claims

1. A peritoneal membrane protecting agent comprising a pyridoxine or a salt thereof, as an active ingredient.

2. The peritoneal membrane protecting agent of claim 1 wherein the pyridoxine is pyridoxamine.

3. The peritoneal membrane protecting agent of claim 1 or 2 which is is used for treating and/or preventing peritoneal dysfunction or ultrafiltration failure in a peritoneal dialysis patient.


SEQUENCE LISTING

<110>  Tokai University

<120>  A protective agent for peritoneal membrane

<130>  A61374A

<160>  6

<210>  1

<211>  21

<212>  DNA

<213>  Artificial Sequence

<220>

<223>  Description of Artificial Sequence: Synthetic DNA

<400>  1

actggaccct ggctttactg c                                21

<210>  2

<211>  22

<212>  DNA

<213>  Artificial Sequence

<220>

<223>  Description of Artificial Sequence: Synthetic DNA

<400>  2

ttggtgaggt ttgatccgca tg                               22

<210>  3

<211>  21

<212>  DNA

<213>  Artificial Sequence

<220>

<223>  Description of Artificial Sequence: Synthetic DNA

<400>  3

caagcagaag agagaggagt t 21
<210> 4
<211> 21
<212> DNA
<213> Artificial Sequence
<220>
<223> Description of Artificial Sequence: Synthetic DNA
<400> 4
tcaagctctt agcagacatt g 21
<210> 5
<211> 30
<212> DNA
<213> Artificial Sequence
<220>
<223> Description of Artificial Sequence: Synthetic DNA
<400> 5
gtgtgatggt gggtatgggt cagaaggact 30
<210> 6
<211> 30
<212> DNA
<213> Artificial Sequence
<220>
<223> Description of Artificial Sequence: Synthetic DNA
<400> 6
atggcatgag ggagcgcgta accctcatag 30